# EUROPEAN PATENT SPECIFICATION

(11) **EP 2 407 203 B1**
(45) Date of publication and mention of the grant of the patent: **18.09.2019**
(21) Application number: 11007488.7
(22) Date of filing: 11.02.2005
(51) Int. Cl.: A61M 31/00, A61F 2/88, A61K 9/00, A61L 27/00

(54) **Implantable drug delivery device**
Implantierbare Arzneimittelabgabevorrichtung
Dispositif d'administration de médicaments implantable

(30) Priority: 13.02.2004 US 544663 P
(43) Date of publication of application: 18.01.2012
(62) Divisional of application: 08016718.2
(73) Proprietor: Shanghai MicroPort Medical (Group) Co., Ltd., Shanghai 201203 (CN)
(72) Inventor: Shanley, John F., Emerald Hills, CA 94062-4072 (US)
(74) Representative: Diehl & Partner GbR

(56) References cited:
- WO-A-2004/004602
- WO-A1-01/01890
- WO-A1-02/060506
- US-A- 6 071 305

## Description

### Background

Many diseases of localized regions in the body are treated by systemic delivery of therapeutic agents. Unfortunately, the systemic delivery of therapeutic agents often is ineffective, inefficient, and/or results in undesirable side effects. For example, cancer treatments often involve systemic administration of chemotherapeutic agents which result in serious side effects to the patient.

Targeted local delivery of a drug to a particular tissue or organ to be treated would provide better efficacy with lower systemic toxicity if a local drug delivery device was available for delivery of a sufficient amount of drug over an extended period of time.

Known implantable local drug delivery systems include implantable osmotic pumps, injectable biodegradable polymers containing drug, plastic drug pellets, and drug containing microspheres. The relatively large size of many of these devices limits their use to a few applications. The smaller systems including biodegradable polymers and microspheres may tend to migrate within the body and may not be able to deliver many types of drugs because of incompatibility of the drugs with the particular polymers used in these systems.

Coated drug delivery devices have been proposed, however, the drug coating can be scraped or flaked off during delivery of the device. In addition, the amount of drug which can be delivered by a coating is limited by the surface area of the device.

Thus, it would be desirable to provide a new implantable drug delivery device for controlled delivery of drug over an extended administration period without the drawbacks of the known systems.

### Summary of the Invention

The present invention relates to a small size implantable drug delivery device which can deliver a therapeutic agent in a controlled manner over an extended period of time from one or more filaments or wires which can be shaped to accommodate a particular target tissue organ within the body, in accordance with claim 1.

### Brief Description of the Drawings

The invention will now be described in greater detail with reference to the preferred embodiments illustrated in the accompanying drawings, in which like elements bear like reference numerals, and wherein:
FIG. 1 is a perspective view of a drug delivery device formed from a wire.
FIG. 2 is a perspective view of a coil drug delivery device.
FIG. 3 is a perspective view of a drug delivery device in the form of a wire mesh.
FIG. 4 is a perspective view of a drug delivery device formed from a wire with multi-directional holes.
FIG. 5 is a perspective view of a drug delivery device formed from a wire with holes and ductile hinges.
FIG. 6 is a perspective view of a drug delivery device formed from a ribbon shaped wire.
FIG. 7 is a perspective view of a drug delivery device in the form of a wire with a hollow interior for delivery of additional drug.
FIG. 8 is a schematic perspective view of a system for filling the holes in the drug delivery device of FIG. 1.
FIG. 9 is a schematic perspective view of another system for filling the holes in a hollow drug delivery device.

### Detailed Description

The terms "drug" and "therapeutic agent" are used interchangeably to refer to any therapeutically active substance that is delivered to a bodily conduit of a living being to produce a desired, usually therapeutic, effect.

The terms "matrix" and "biocompatible matrix" are used interchangeably to refer to a medium or material that, upon implantation in a subject, does not elicit a detrimental response sufficient to result in the rejection of the matrix. The matrix may contain or surround a therapeutic agent, and/or modulate the release of the therapeutic agent into the body. A matrix is also a medium that may simply provide support, structural integrity or structural barriers. The matrix may be polymeric, non-polymeric, hydrophobic, hydrophilic, lipophilic, amphiphilic, and the like. The matrix may be bioresorbable or non-bioresorbable.

The term "solid" when referring to a therapeutic agent is used to refer to either a solid or gel form of a therapeutic agent which may be incorporated in a matrix or in any other substantially non-flowable form at body temperature which allows the agent to be retained within holes.

The term "bioresorbable" refers to a matrix, as defined herein, that can be broken down by either chemical or physical process, upon interaction with a physiological environment. The matrix can erode or dissolve. A bioresorbable matrix serves a temporary function in the body, such as drug delivery, and is then degraded or broken into components that are metabolizable or excretable, over a period of time from minutes to years, preferably less than one year, while maintaining any requisite structural integrity in that same time period.

The term "holes" includes both through holes and recesses of any shape.

The term "pharmaceutically acceptable" refers to the characteristic of being non-toxic to a host or patient and suitable for maintaining the stability of a therapeutic agent and allowing the delivery of the therapeutic agent to target cells or tissue.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{W} greater than about 3000 and preferably greater than about 10,000 and a M_{W} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclo dextrin sulfo butyl ethers; polypeptides, and proteins such as polylysine, polyglutamic acid, albumin; polyanhydrides; polyhydroxy alkonoates such as polyhydroxy valerate, polyhydroxy butyrate, and the like.

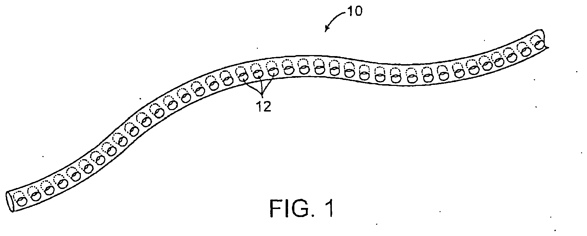

FIG. 1 illustrates an implantable drug delivery device in the form of a wire or filament shaped device having holes for containing a therapeutic agent to be delivered to a target organ or tissue. The drug delivery device 10 of FIG. 1 includes a plurality of holes 12 which have been laser cut or otherwise formed to extend through the thickness of the wire. Thus, each of the holes 12 has two open ends for drug delivery and an interior reservoir for containing one or more therapeutic agents.

The drug delivery device 10 of FIG. 1 is illustrated in a wave shape, however, many other shapes are also useful and will be described further below. The wave shaped device 10 can be implanted in body lumens, tissue, organs, or body cavities to deliver the therapeutic agent locally at a desired delivery site over a selected time period which can be from several hours to many months. The extended release of a wide variety of drugs is achieved in most cases by providing the drug in a biocompatible polymer matrix which may be either bioresorbable or non-bioresorbable. The release profile can be tailored to the particular drug and application by the selection of and the arrangement of the polymer matrix and drug in the holes. The selected release profile may be linear, pulsatile, first order, or increase or decreasing in rate.

In one example, the drug delivery device 10 of FIG. 1 may be used for delivery of a therapeutic agent used for expanding bodily lumens, such as collateral arteries. The drug delivery device 10 can be formed of a shape memory or spring metal material wire with a memory or set shape in the form of a large wave, spiral, coil z-shape, or other shape. This device 10 can be straightened and inserted into a tube or otherwise constrained for delivery to a collateral artery. Upon delivery, the tube or other constraining device is removed and the device is seated in the artery with the wave or spiral shape pressing against the walls of the artery. Due to the shape memory or spring metal material, the device 10 will tend to follow the expanding shape of the artery as the artery is expanded by a therapeutic agent and will continue to deliver the agent to the artery. The therapeutic agents which may be particularly suitable for expansion of collateral arteries include vasodilators, NO donors, or other drugs which act to cause endothelial cells to draw away from the drug.

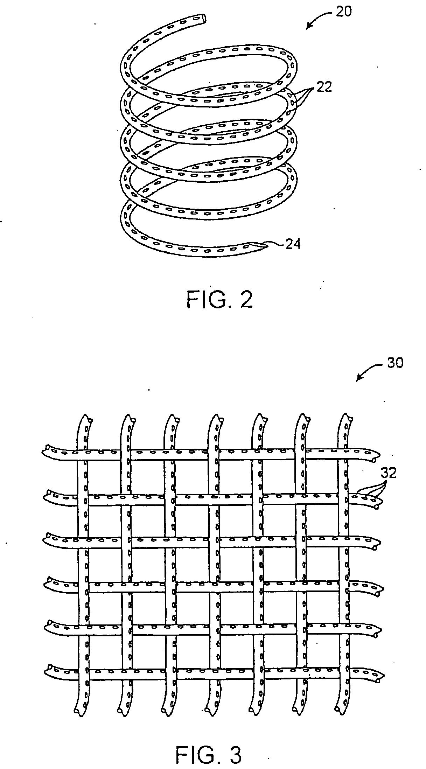

FIG. 2 illustrates a wire drug delivery device in the shape of a coil 20 having a plurality of holes 22 for containing a beneficial agent. The coil 20 may include a sharpened tip 24 for penetrating tissue. The coil 20 may be inserted into tissue by penetrating the tissue with the tip 24 and rotating the coil to insert the coil into tissue. The coil 20 may be used to deliver drugs locally to a tissue or organ in areas where coated drug delivery devices, injectable polymers, pellets, microspheres, or osmotic devices are presently used. For example, the coil 20 can be used to deliver chemotherapeutic agents to tumors, tissue regenerating agents, beta blockers, vasodilators, diaretics, antibiotics and the like.

FIG. 3 illustrates a wire drug delivery device in which the wire has been formed into a wire mesh 30. The wire mesh 30 is formed from wire in which holes 32 have been cut, such as with a laser. The drug may be loaded into the holes before or after forming the wire into the mesh 30. The wire mesh 30 can be used to wrap around particular organs or tissue to deliver drug locally to the target tissue.

The embodiments of FIGS. 1-3 have been illustrated with holes 12, 22, 32 extending all the way through the wires in a single direction. Alternatively, the holes in any of the embodiments discussed herein may be cut in more than one direction. The wire may also be formed with holes in more than two directions.

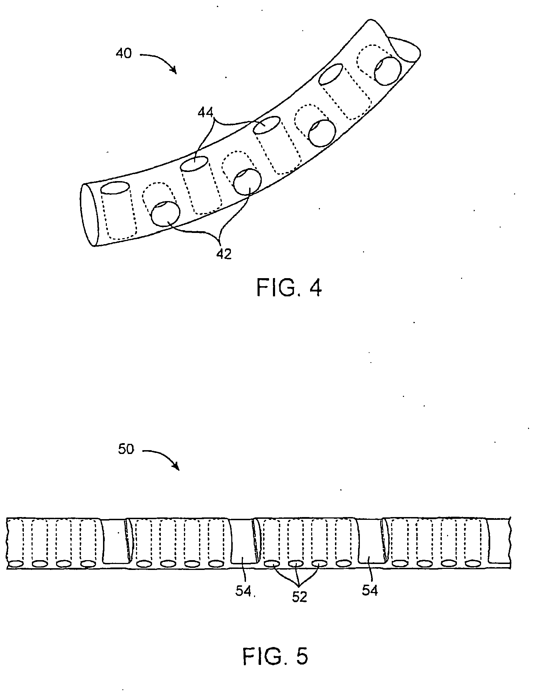

FIG. 4 illustrates a wire 40 having first holes 42 in one direction and second holes 44 in a generally perpendicular direction. The wire may also be formed with holes in more than two directions. Although the holes shown herein have been illustrated generally perpendicular to the axis of the wire, the holes may also be angled with respect to the wire axis.

The holes, when round, are generally selected to have a diameter which is about 10% to about 80% of the diameter of the wire, preferably about 25% to about 60%. This will allow the wire to maintain structural integrity and reduce kinking. The holes can have volumes ranging from about 0.1 nanoliters to about 50 nanoliters. The wire generally has a widest cross sectional dimension of about 0.006 mm to 0.04 mm.

FIG. 5 illustrates an alternative embodiment of a wire drug delivery device 50 having a plurality of holes 52 and a plurality of ductile hinges 54. The plurality of ductile hinges 54 may be formed in the same laser cutting operation as the holes 52 and provide a location for preferential bending of the wire. The hinges 54 allow the wire to be easily bent to a shape to accommodate a particular tissue or organ. For example, when the wire 50 is formed into a mesh, the mesh will be easily formed around an organ and will hold its shape due to the ductile hinges 54. A further discussion of some examples of ductile hinges can be found in U.S. Patent No. 6532065. The ductile hinges 54 can be formed all in one direction as shown in FIG. 5 or can be formed to provide for bending in more than one direction. When the wire 50 is bent at the locations of the ductile hinges 54, the holes 52 are non-deforming and can be filled with materials which would crack, extrude, or change delivery profile if deformed.

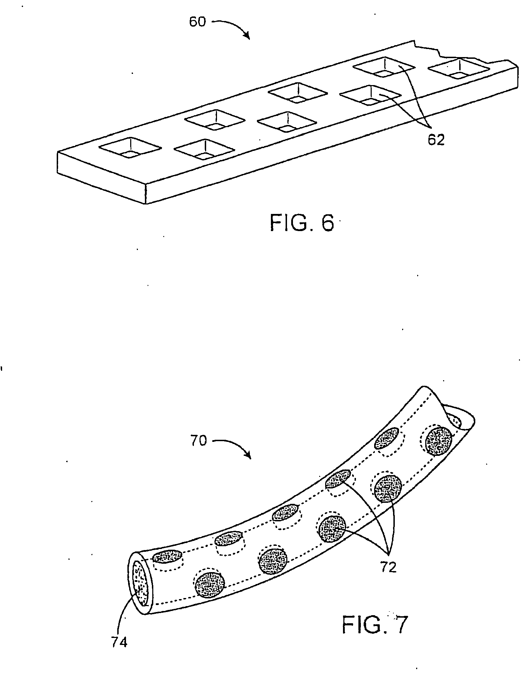

FIG. 6 illustrates an alternative embodiment of a rectangular or ribbon shaped wire 60 with a plurality of substantially square holes 62. The rectangular shaped wire 60 can be used as a coil, filament, mesh, or other shape drug delivery device depending on the application.

FIG. 7 illustrates a drug delivery device 70 in the form of a hollow wire with a plurality of holes 72. In the example shown, the hollow wire 70 has a central lumen 74 containing a first agent while the holes 72 contain a second agent. This configuration allows the sequential delivery of two agents. Alternatively, the same agent or agents may be contained in the holes 72 and the lumen 64 allowing delivery of larger amounts of agent. The first agent may be delivered to the lumen 74 as a filament, such as a polymer filament, as a liquid, or as a flowable material. Examples of first and second agents include first and second angiogenic agents which are programmed to be delivered at different times to promote different stages of the process of angiogenesis. Angiogenic agents include VEG-A, VEG-145, VEGF, FGF, HGF, Ang1, Ang2, insulin-like growth factor, and the like.

The structures described in FIGS. 1-7 can be used to deliver one agent or a plurality of agents. For example, a first agent may be provided in the same holes with a second agent in different layers, concentration gradients, regions, or in mixed configurations. Alternatively, first and second agents can be provided in interspersed holes on the same wire. For embodiments using multiple wires, such as the wire mesh, different agents may be provided in different wires. Further, different agents may be placed in different areas (i.e. different ends) of the same wire.

When the biocompatible matrix containing therapeutic agent is disposed within the holes in the wire structures of the present invention to form a plurality of drug delivery reservoirs, the holes may be partially or completely filled with matrix containing the therapeutic agent. The holes may also be filled with one or more protective or separating layers or areas of matrix which act to control direction and/or timing of the release of the therapeutic agent. For example in the mesh embodiment of FIG. 3, a barrier layer may be provided at one side of the holes to provide directional delivery of the drug to one side of the mesh.

Individual chemical compositions and pharmacokinetic properties can be imparted to different areas of the matrix. Each of the areas of the matrix may include one or more agents in the same or different proportions from one area to the next. Further combinations of two or more agents with independent concentration gradients can provide a range of controlled release kinetic profiles of the agents from the matrix.

The matrix may be solid, porous, or filled with other drugs or excipients. The agent may be in one or both of a solid solution morphology, and a solid emulsion morphology. The agents may be homogeneously disposed or heterogeneously disposed in different areas of the matrix.

### Therapeutic Agents

Some of the therapeutic agents for use with the present invention include, but are not limited to, immunosuppressants, antibiotics, antilipid agents, antiinflammatory agents, chemotherapeutic agents, antineoplastics, antiplatelets, angiogenic agents, anti-angiogenic agents, vitamins, antimitotics, metalloproteinase inhibitors, NO donors, estradiols, anti-sclerosing agents, and vasoactive agents, endothelial growth factors, estrogen, beta blockers, AZ blockers, hormones, statins, insulin growth factors, antioxidants, membrane stabilizing agents, calcium antagonists, retenoid, antineoplastics, antiangiogenics, antirestenotics, anti-thrombotics, such as heparin, antiproliferatives, such as paclitaxel and Rapamycin, tissue regenerating agents, vasodilators, and diaretics alone or in combinations with any therapeutic agent mentioned herein. Therapeutic agents also include peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense, oligonucleotides, monoclonal antibodies, platelets, prions, viruses, bacteria, and eukaryotic cells such as endothelial cells, stem cells, ACE inhibitors, monocyte/macrophages or vascular smooth muscle cells to name but a few examples. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are delivered into the holes in the wires. Therapeutic agents may also be radioactive isotopes or agents activated by some other form of energy such as light or ultrasonic energy, or by other circulating molecules that can be systemically administered. Therapeutic agents may perform multiple functions including modulating angiogenesis, restenosis, cell proliferation, thrombosis, platelet aggregation, clotting, and vasodilation. Anti-inflammatories include non-steroidal anti-inflammatories (NSAID), such as aryl acetic acid derivatives, e.g., Diclofenac; aryl propionic acid derivatives, e.g., Naproxen; and salicylic acid derivatives, e.g., aspirin, Diflunisal. Anti-inflammatories also include glucocoriticoids (steroids) such as dexamethasone, prednisolone, and triamcinolone. Anti-inflammatories may be used in combination with other drugs to mitigate the reaction of the tissue to the drug and implant.

Some of the agents described herein may be combined with additives which preserve their activity. For example additives including surfactants, antacids, antioxidants, and detergents may be used to minimize denaturation and aggregation of a protein drug, such as insulin. Anionic, cationic, or nonionic detergents may be used. Examples of nonionic additives include but are not limited to sugars including sorbitol, sucrose, trehalose; dextrans including dextran, carboxy methyl (CM) dextran, diethylamino ethyl (DEAE) dextran; sugar derivatives including D-glucosaminic acid, and D-glucose diethyl mercaptal; synthetic polyethers including polyethylene glycol (PEO) and polyvinyl pyrrolidone (PVP); carboxylic acids including D-lactic acid, glycolic acid, and propionic acid; detergents with affinity for hydrophobic interfaces including n-dodecyl-p-D-maltoside, n-octyl-β-D-glucoside, PEO-fatty acid esters (e.g. stearate (myrj 59) or oleate), PEO-sorbitan-fatty acid esters (e.g. Tween 80, PEO-20 sorbitan monooleate), sorbitan-fatty acid esters (e.g. SPAN 60, sorbitan monostearate), PEO-glyceryl-fatty acid esters; glyceryl fatty acid esters (e.g. glyceryl monostearate), PEO-hydrocarbon-ethers (e.g. PEO-10 oleyl ether; triton X-100; and Lubrol. Examples of ionic detergents include but are not limited to fatty acid salts including calcium stearate, magnesium stearate, and zinc stearate; phospholipids including lecithin and phosphatidyl choline; CM-PEG; cholic acid; sodium dodecyl sulfate (SDS); docusate (AOT); and taumocholic acid.

### Filling Systems

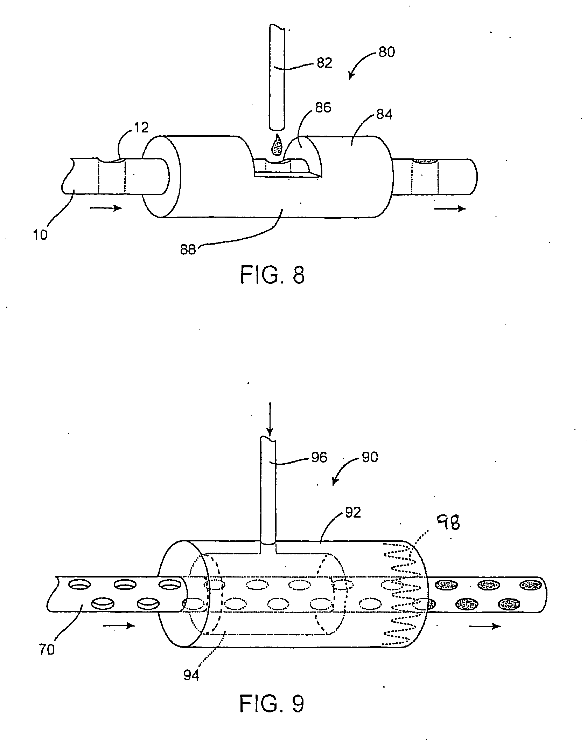

FIG. 8 illustrates one example of a system 80 for filling holes in a drug delivery device. The system 80 can operate in a continuous manner to fill multiple holes along the length of a wire as the wire passes from a spool through the filling system and onto a takeup spool or to another finishing or forming system.

In FIG. 8, the drug delivery device illustrated is the wire device 10 of FIG. 1 having holes formed in one direction, however, other devices can also be filled in this manner. The system 80 includes a dispenser 82 for delivering droplets and a bushing 84 for holding the wire 10 during filling of the holes 12. The dispenser may be any dispenser capable of delivering droplets or filaments of less than a nanoliter, such as a piezoelectric dispenser. In FIG. 8, as the wire 10 passes through the bushing 84 the holes are individually filled with the agent which solidifies in the holes. The bushing 84 includes an open window 86 through which the agent can be dispensed into the holes. The window 86 can also allow the visualization of the hole by a visualization system including one or more cameras. The bushing 84 also includes a closed bottom 88 which blocks the bottom side of the holes and retains the dispensed agent in the holes until it is sufficiently solidified. The bushing includes sealing elements where necessary which may include rubber coatings, resilient tubing, or the like.

In the event that the holes to be filled are provided in multiple directions in the wire, such as shown in the embodiment of FIG. 4, the system 80 of FIG. 8 may be used to fill the holes in one direction followed by an additional system for filling the holes in the other direction. Alternately, the wire may be translated and rotated in the bushing to fill holes in multiple directions.

The agent which is delivered to the holes by the dispenser 82 may be dispensed as a combination of drug, polymer, and a solvent. The delivery steps can be repeated to provide regions of differing agent combinations within the holes which provide controlled release of the agent. The solvent can be evaporated by heating to achieve a solid inlay of the agent. Alternately, the agent may be delivered as a hot melt without solvent or with minimal solvent. In the hot melt example, the bushing 84 can be cooled to provide a cool bottom at the surface of the hole which quickly solidifies the hot melt.

In one embodiment multiple systems of FIG. 8 are arranged in series with optional heat transfer stages in between. In this way multiple layers of agent may be deposited in the holes with the agent being solidified by the intermediate heat transfer stages. In another alternative embodiment, a discontinuous filling system may be used to fill segments of the wire retained in a fixturing device.

Examples of some dispensers, visualization systems, and control systems useful in the present invention are described in U.S. Patent Publication No. 2004/0127976A1.

FIG. 9 illustrates an alternative system 90 for filling holes by a continuous molding process. The filling system 90 includes a mold 92 with an interior mold cavity 94 into which liquefied agent is delivered through an agent inlet 96. The wire 70, such as the wire of FIG. 7, is passed through the mold cavity 94 and any openings in the wire become filled with the liquid agent. The liquefied agent may be an agent, polymer, solvent composition or a hot melt agent, and polymer composition. When a hot melt agent is used, the mold 92 may include a cooling zone 98 having cooling coils through which the filled wire 70 passes to at least partially solidify the agent before the wire exits the mold. The system of FIG. 9 is particularly advantageous for filling the hollow wire 70 shown in FIG. 7 because the hollow lumen within the wire serves as a pressure relief eliminating a need for a pressure relief in the mold. The system of FIG. 9 may also be used for filling wires of other shapes and configurations.

## Claims

1. A drug delivery device having holes in it, wherein the device is in the form of at least one hollow wire (70) having a central lumen (74) and the holes are through holes (72),
**characterized in that**
the central lumen (74) is filled with a first agent and the through holes (72) are filled with a second agent, and the second-agent-filled through holes (72) are provided in two or more directions in the wire (70), wherein the first agent is different than the second agent.

2. The device according to claim 1, wherein the through holes (72) communicate the lumen (74) with the outside.

3. The device according to claim 1, wherein the at least one wire is formed in a coil.

4. The device according to claim 1, wherein the at least one wire is formed in a wire mesh.

5. The device according to claim 1, wherein the first agent comprises a chemotherapeutic agent and a polymer matrix.

6. The device according to claim 1, wherein the second agent comprises a chemotherapeutic agent and a polymer matrix.

7. The device according to claims 5 or 6, wherein the polymer matrix is biodegradable.

8. The device according to claim 5 or 6, wherein the polymer matrix is non-biodegradable.

## Patentansprüche

1. Wirkstoff-Abgabe-Vorrichtung mit Löchern darin, wobei die Vorrichtung in der Gestalt wenigstens eines hohlen Drahtes (70) mit einem zentralen Lumen (74) vorliegt, und die Löcher Durchgangslöcher (72) sind,
**dadurch gekennzeichnet, dass**
das zentrale Lumen (74) mit einem ersten Agens gefüllt ist und die Durchgangslöcher (72) mit einem zweiten Agens gefüllt sind, und die mit dem zweiten Agens gefüllten Durchgangslöcher (72) in zwei oder mehr Richtungen in dem Draht (70) bereitgestellt sind, wobei das erste Agens von dem zweiten Agens verschieden ist.

2. Vorrichtung gemäß Anspruch 1, wobei die Durchgangslöcher (72) das Lumen (74) mit dem Äußeren verbinden.

3. Vorrichtung gemäß Anspruch 1, wobei der wenigstens eine Draht als Spirale geformt ist.

4. Vorrichtung gemäß Anspruch 1, wobei der wenigstens eine Draht als Drahtgitter geformt ist.

5. Vorrichtung gemäß Anspruch 1, wobei das erste Agens ein chemotherapeutisches Agens und eine Polymermatrix beinhaltet.

6. Vorrichtung gemäß Anspruch 1, wobei das zweite Agens ein chemotherapeutisches Agens und eine Polymermatrix beinhaltet.

7. Vorrichtung gemäß Anspruch 5 oder 6, wobei die Polymermatrix bioabbaubar ist.

8. Vorrichtung gemäß Anspruch 5 oder 6, wobei die Polymermatrix nicht bioabbaubar ist.

## Revendications

1. Dispositif d'administration de médicaments ayant des trous dans celui-ci, dans lequel le dispositif est sous la forme d'au moins un fil creux (70) ayant une lumière centrale (74) et les trous sont des trous traversants (72),
**caractérisé en ce que**
la lumière centrale (74) est remplie d'un premier agent et les trous traversants (72) sont remplis d'un second agent, et les trous traversants remplis du second agent (72) sont prévus dans deux directions ou plus dans le fil (70), dans lequel le premier agent est différent du second agent.

2. Dispositif selon la revendication 1, dans lequel les trous traversants (72) font communiquer la lumière (74) avec l'extérieur.

3. Dispositif selon la revendication 1, dans lequel l'au moins un fil est formé dans une bobine.

4. Dispositif selon la revendication 1, dans lequel l'au moins un fil est formé dans un treillis métallique.

5. Dispositif selon la revendication 1, dans lequel le premier agent comprend un agent chimiothérapeutique et une matrice polymère.

6. Dispositif selon la revendication 1, dans lequel le second agent comprend un agent chimiothérapeutique et une matrice polymère.

7. Dispositif selon les revendications 5 ou 6, dans lequel la matrice polymère est biodégradable.

8. Dispositif selon les revendications 5 ou 6, dans lequel la matrice polymère est non biodégradable.
